# EUROPÄISCHE PATENTSCHRIFT

(11) **EP 1 573 328 B1**
(45) Veröffentlichungstag und Bekanntmachung des Hinweises auf die Patenterteilung: **16.10.2013**
(21) Anmeldenummer: 03795751.1
(22) Anmeldetag: 15.12.2003
(51) Int. Cl.: G01N 33/543, C12Q 1/68

(54) **BIO-CHIP**
BIOCHIP
BIOPUCE

(30) Priorität: 19.12.2002 DE 10259821
(43) Veröffentlichungstag der Anmeldung: 14.09.2005
(73) Patentinhaber: Siemens Aktiengesellschaft, 80333 München (DE)
(72) Erfinder: STANZEL, Manfred, 91056 Erlangen (DE); GUMBRECHT, Walter, 91074 Herzogenaurach (DE)
(86) Internationale Anmeldenummer: PCT/DE2003/004137
(87) Internationale Veröffentlichungsnummer: WO 2004/057335

(56) Entgegenhaltungen:
- WO-A-00/62047
- WO-A-02/41992
- DE-A- 19 610 115
- US-A- 5 534 132
- PROUDNIKOV D ET AL: "IMMOBILIZATION OF DNA IN POLYACRYLAMIDE GEL FOR THE MANUFACTURE OF DNA AND DNA-OLIGONUCLEOTIDE MICROCHIPS" ANALYTICAL BIOCHEMISTRY, ACADEMIC PRESS, SAN DIEGO, CA, US, Bd. 259, 1998, Seiten 34-41, XP002928888 ISSN: 0003-2697
- MARQUETTE CHRISTOPHE A ET AL: "Regenerable immunobiosensor for the chemiluminescent flow injection analysis of the herbicide 2,4-D" 7. Februar 2000 (2000-02-07), TALANTA, VOL. 51, NR. 2, PAGE(S) 395-401 , XP002279239 ISSN: 0039-9140 Zusammenfassung

## Beschreibung

Die Erfindung betrifft einen Bio-Chip, insbesondere DNA-Chip, gemäß dem Patentanspruch 1.

Bio- bzw. DNA-Chips umfassen einen Flachträger, auf dessen einer Seite wenigstens ein Spot-Array, also eine rasterartige Anordnung von Analysepositionen vorhanden ist. Die Spots enthalten auf der Trägeroberfläche immobilisierte Sonden- oder Fängermoleküle, beispielsweise Oligonucleotide. In einer auf einen Spot applizierten Analytlösung enthaltene Zielmoleküle, beispielsweise DNA-Bruchstücke koppeln an die Fängermoleküle an. Die Umwandlung solcher Koppel- bzw. Bindungsereignisse in detektierbare Signale erfolgt mit optischen, piezoelektrischen, elektrochemischen, kalorimetrischen oder impedanzspektroskopischen Methoden.

Bei einem aus DE 196 10 115 C2 bekannten impedanzspektroskopisch auslesbaren DNA-Chip ist auf einer Sensorfläche eine interdigitale Elektrodenanordnung vorhanden, wobei Fängermoleküle auf den Elektroden und den zwischen den Elektroden angeordneten Flächen immobilisiert sind. Die Ankopplung von Zielmolekülen an die Fängermolekülen führt z.B. aufgrund von Ladungsänderungen zu einer Änderung des von den Elektroden erzeugten elektrischen Wechselfeldes bzw. allgemein zu einer Änderung einer elektrischen Eigenschaft in der Umgebung der Elektroden, z.B. der Impedanz. Eine Messung einer Impedanzänderung lässt sich mit einer beispielsweise 2-poligen interdigitalen Elektrodenanordnung durchführen, bei der die Elektroden aus mehreren Teilelektroden gebildet sind.

Problematisch bei letzter Art der Detektion von Bindungsereignissen ist, dass sich die Abmessungen der Elektrodenstrukturen um Größenordnungen von molekularen Dimensionen unterscheiden. Mit noch vertretbarem technischen Aufwand lassen sich Elektroden herstellen, mit deren Breite und Abstand zusammengenommen einen Wert L (= Breite + Abstand) von etwa 2 bis 20 µm und eine Höhe von etwa 0,1 bis 0,5 µm aufweisen.

Der impedanzspektroskopisch erfassbare Bereich des elektrischen Feldes einer solchen Elektrodenanordnung erstreckt sich etwa 1 bis 5 L (= 2 bis 100 µm) über die Trägeroberfläche bzw. die von der Elektrodenanordnung aufgespannte Planebene hinaus. Dagegen hat ein beispielsweise 100 Basenpaare aufweisendes Fängermolekül eine Länge von nur etwa 30 nm. Entsprechend gering ist der Einfluss von Bindungsereignissen in einer auf der Sensorfläche bzw. den Elektroden immobilisierten monomolekularen Schicht von Fängermolekülen auf das elektrische Feld, insbesondere dann, wenn nur wenige Bindungsvorgänge stattfinden. In der Veröffentlichung "Nanoscaled interdigitated electrode arrays for biochemical sensors", P. van Gerwen et al, Sensors and Actuators B 49, 1998, 72 - 80 wird zur Lösung des angesprochenen Problems eine Annäherung der Dimensionen von Elektrodenstrukturen an die Dimensionen von DNA-Zielmolekülen vorgeschlagen, wobei Elektrodenstrukturen mit Teilelektroden angestrebt werden, deren Breiten und gegenseitigen Abstände etwa im Bereich von 250 bis 500 nm liegen. Solche Dimensionen sind jedoch mit einem erhöhten Herstellungsaufwand verbunden.

Weiterhin ist aus der WO 98/19153 A1 ein Sensor für biochemische Anwendungen bekannt, bei dem Elektroden vorhanden sind, die in ein leitfähiges Polymer eingebettet sind. Das leitfähige Polymer ist dabei in Kontakt mit dem Analyten, in dem durch Wechselstrombeeinflussung ein biochemischer Prozess stattfindet. Dabei werden Prozessänderungen über das leitfähige Polymer als Impedanzänderungen zum Elektrodensystem weitergegeben und erfasst.

Die Sensitivität eines solchermaßen aufgebauten Sensorchips ist problematisch. Außerdem ist das Einbetten bzw. Beschichten der Elektroden n ein leitfähiges Polymer aufwendig, so dass der beschriebene Biosensor nicht praxistauglich ist.

Das Dokument WO00/62047 beschreibt einen DNA-Chip mit einem Flachträger und einem darauf angeordneten Array von Fängermolekülen enthaltenden Spots, wobei jedem Spot eine Mikroelektroden-Anordnung zur Detektion von Bindungsereignissen zwischen den Fängermolekülen und mittels einer Analytlösung applizierten Zielmolekülen zugeordnet ist, wobei die Elektrodenanordnung zumindest teilweise in eine für Zielmoleküle durchlässige hydrophile Reaktionsschicht eingebettet ist, in der immobilisierte Fängermoleküle enthalten sind

Ausgehend vom Stand der Technik ist es Aufgabe der Erfindung, einen kostengünstig herstellbaren, impedanzspektroskopisch auslesbaren DNA-Chip mit verbesserter Sensitivität vorzuschlagen.

Die Aufgabe ist erfindungsgemäß durch die Merkmale des Patentanspruchs 1 gelöst. Weiterbildungen sind in den Unteransprüchen angegeben.

Bei der Erfindung ist die Elektrodenanordnung zumindest teilweise in eine für Zielmoleküle durchlässige hydrophile Reaktionsschicht eingebettet, in der immobilisierte Fängermoleküle dreidimensional verteilt sind. Die Reaktionsschicht ist dabei so dimensioniert, dass sie von dem überwiegenden Teil des von der Elektrodenanordnung erzeugten elektrischen Feldes bzw. von deren impedanzspektroskopischen Erfassungsbereich durchdrungen ist.

Ein wesentlicher Vorteil eines erfindungsgemäßen Biochips besteht darin, dass innerhalb der Reaktionsschicht eine wesentlich größere Anzahl von Fängermolekülen angeordnet werden kann als in_einer monomolekularen Schicht auf der Trägeroberfläche und auf den Oberflächen der Elektroden. Hinzu kommt aber noch, dass die Abmessung der Reaktionsschicht an den vom elektrischen Feld bzw. von dessen Feldlinien durchdrungenen Raum angepasst ist, so dass eine große Anzahl bzw. eine hohe Konzentration von Fängermolekülen mit etwa homogener Verteilung innerhalb des genannten Erfassungsbereiches vorhanden ist. Die Folge ist eine weitaus größere Beeinflussung des elektrischen Feldes bzw. des impedanzspektroskopischen Erfassungsbereiches der Elektrodenanordnung. Ein derart ausgestalteter DNA-Chip weist eine entsprechend größere Messempfindlichkeit bzw. Sensitivität auf.

Die Dicke der Reaktionsschicht sollte vorteilhafterweise maximal 100 *µ*m betragen. Für die Praxis darf die Dicke der Reaktionsschicht aber nicht zu groß gewählt werden, weil daraus zu lange Diffusionswege und damit verbunden zu lange Reaktionszeiten für den Transport der Zielmoleküle zu den Fängermolekülen resultieren würden.

Bei Elektrodenbreiten im Bereich von etwa 1 *µ*m und ebensolchen Abständen beträgt die Dicke der Reaktionsschicht zwischen 2 und 10 um, beispielsweise etwa 3 *µ*m bei einem Zwei-Pol-Mikroelektrodensystem und etwa 7 *µ*m bei einem Vier-Pol-Mikroelektrodensstem.

Die Reaktionsschicht weist eine Dicke auf, die etwa im Bereich von 1 - 5 L liegt, wobei L die Summe aus der Elektrodenbreite und dem Elektrodenabstand ist. Dadurch ist gewährleistet, dass einerseits ein Bereich des elektrischen Feldes mit relativ hoher Feldliniendichte zur Detektion von Bindungsereignissen genutzt wird und andererseits die Dicke der Reaktionsschicht nicht so groß ist, dass sie das Eindiffundieren von Zielmolekülen und Reaktanten behindert.

Mit einer bis etwa 95°C thermisch stabilen Reaktionsschicht ist ein DNA-Chip der in Rede stehenden Art für PCR-Reaktionen anwendbar. Thermisch stabil soll dabei bedeuten, dass die Reaktionsschicht auch bei der genannten Temperatur derart beschaffen ist, dass sie sich nicht auflöst, dass sie Fängermoleküle festhält, dass in ihr Reaktionen zwischen Ziel- und Fängermolekülen ungehindert stattfinden können und dass sie auch ihre sonstigen Eigenschaften im wesentlichen beibehält. Bei einer weiteren bevorzugten Ausgestaltung enthält die Reaktionsschicht Polymere mit Kopplungsgruppen, an die Fängermoleküle kovalent gebunden sind. Dadurch ist sicher gewährleistet, dass Bindungspaare aus Ziel- und Fängermolekülen bei Spülvorgängen während des Analysenganges in der Reaktionsschicht zurückgehalten werden. Eine besonders geeignete Reaktionsschicht besteht aus einem Hydrogel. Hydrogele bilden ein wässriges Milieu in mechanisch stabiler Form, das einen Stoffaustausch mit einem überwiegend wässrigen Analyten erlaubt. Als besonders geeignet haben sich radikalisch vernetzbare Hydrogele auf Acrylamidbasis mit Maleinsäureanhydrid und/oder Glycidyl(meth)acrylat als Kopplungsgruppen erwiesen.

Bei einer weiteren bevorzugten Ausführungsform umfasst der Flachträger des DNA-Chips eine Siliziumschicht und eine damit verbundene Isolierschicht, wobei letztere auf ihrer der Siliziumschicht abgewandten Seite die Elektrodenanordnung und die Reaktionsschicht trägt. Bei einer solchen Anordnung lässt sich die elektrische Verschaltung der Elektrodenstruktur mit aus der Si-Halbleitertechnologie bekannten Analog- und Digital-Schaltungen verwirklichen.

Weitere Einzelheiten und Vorteile der Erfindung ergeben sich aus der Figurenbeschreibung von Ausführungsbeispielen anhand der Zeichnung in Verbindung mit den Patentansprüchen. Es zeigen:
- Fig. 1: eine vereinfachte perspektivische Darstellung eines einen Flachträger und ein Spot-Array umfassenden Biochips,
- Fig. 2: einen Querschnitt durch einen Spot entsprechend Linie II-II in Fig. 1, in vergrößerter ausschnittsweiser Darstellung,
- Fig. 3: einen Ausschnitt einer einem Spot zugeordneten Elektrodenanordnung,
- Fig. 4: eine Ausführungsform eines Biochips mit einer 4-poligen Elektrodenanordnung in einer Fig. 2 entsprechenden Darstellung, und
- Fig. 5: die Elektrodenanordnung des Biochips von Fig. 4 in einer Fig. 3 entsprechenden Darstellung.

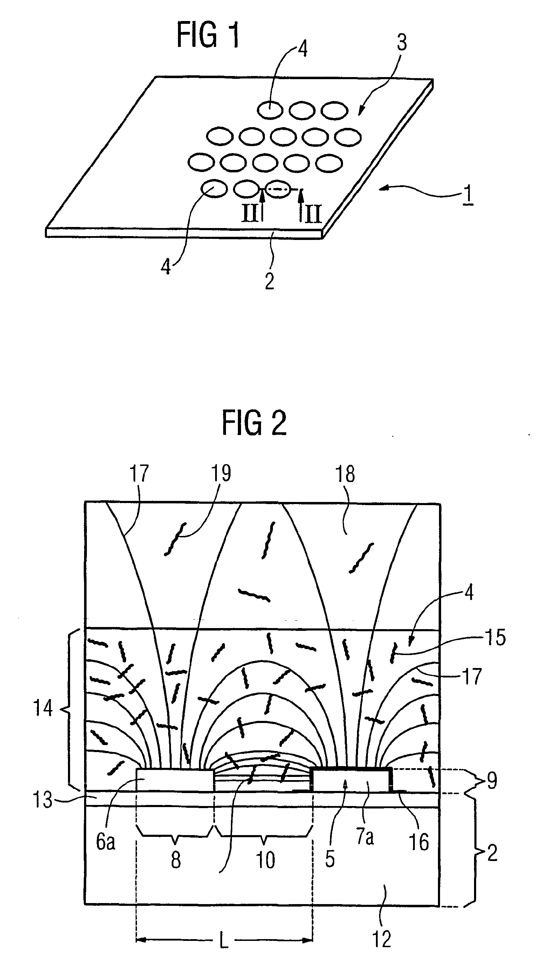

Wie Fig. 1 zeigt, umfasst ein Biochip 1 einen Flachträger 2, auf dessen einer Seite ein Spot-Array 3 aufgebracht ist. Ein Spot 4 enthält immobilisierte Fängermoleküle, beispielsweise Oligonucleotide. Wird auf einen Spot eine Analytlösung mit unbekannten Zielmolekülen aufgebracht, so kommt es bei entsprechender Übereinstimmung in der Basensequenz zu einer Ankopplung des Zielmoleküls an das Fängermolekül. Die durch ein solches Bindungsereignis hervorgerufene Eigenschaftsänderung, z.B. Änderungen des spezifischen Widerstandes oder der Dielektrizitätskonstante, wird mit einer Elektrodenanordnung 5 impedanzspektroskopisch erfasst.

Bei dem Ausführungsbeispiel von Fig. 2 ist eine 2-polige Elektrodenanordnung vorhanden. Diese ist beispielsweise mit Hilfe eines photolithographischen Verfahrens auf den Flachträger 2 aufgebracht. Die Elektrodenanordnung 5 umfasst zwei Elektroden 6, 7, die in Form einer Interdigitalstruktur ausgebildet sind. D.h. jede Elektrode umfasst mehrere streifenförmige parallel zueinander verlaufende Teilelektroden 6a, 7a, die sich jeweils in den Zwischenraum zweier Teilelektroden der jeweils anderen Elektroden hinein erstrecken. Die Teilelektroden 6a, 7a sind durch einen ebenfalls streifenförmigen, sich quer zu den Teilelektroden 6a, 7a streckenden Verbindungsleiter 6b, 7b miteinander verbunden. Die Elektroden 6, 7 sind mit einer Wechselspannung z.B. im Megahertzbereich beaufschlagt. Die Breite 8 der Teilelektroden 6a, 7a beträgt ca. 1 µm, ihre Höhe 9 beträgt etwa 100 bis 500 nm. Zwischen den Teilelektroden 6a, 7a ist ein Abstand 10 von ebenfalls ca. 1 µm vorhanden.

Der Flachträger 2 umfasst eine Siliziumschicht 12 und eine zwischen dieser und den Elektroden 6, 7 angeordnete Isolierschicht 13 z.B. aus Siliziumdioxid oder Siliziumnitrid. Die für die impedanzspektroskopische Messung von Bindungsereignissen erforderlichen elektrischen Verschaltungen und Bauteile sind in herkömmlicher Weise durch eine entsprechende Strukturierung der Siliziumschicht (nicht dargestellt) realisiert. Auf der Isolierschicht 13 ist eine Reaktionsschicht 14 aus einem Hydrogel aufgebracht, welches weiter unten näher beschrieben wird.

In der Reaktionsschicht 14 bzw. dem Hydrogel sind Fängermoleküle 15, die in Fig. 2 überdimensioniert und symbolisch dargestellt sind, eingebettet und homogen verteilt. Ein Fängermolekül mit 300 Basen weist etwa eine Länge von 100 nm auf. Demzufolge hat eine monomolekulare Schicht von Fängermolekülen bei herkömmlichen Biochips allenfalls etwa eine Dicke entsprechend der Linie 16 in Fig. 2. Es ist leicht einsehbar, das eine solche Schicht relativ wenige Fängermoleküle 15 aufnehmen und dementsprechend im Falle von Bindungsereignissen das elektrische Feld nur gering beeinflussen kann. Demgegenüber ist bei einem erfindungsgemäßen Biochip der Fängermoleküle enthaltende und von Feldlinien durchdrungene Reaktionsbereich wesentlich erweitert und bietet Platz für eine um mehrere Zehnerpotenzen größere Anzahl von Zielmolekülen 15. Wird auf ein solcher Art ausgestaltete Spot-Arrays 3 bzw. auf einen Spot 4 eine Analytlösung 18 aufgebracht, so finden die darin enthaltenen Zielmoleküle 19, was in Fig. 2 ebenfalls übertrieben groß und nur symbolisch dargestellt ist, eine wesentlich größere Anzahl möglicher Bindungspartner in Form der Fängermoleküle 15 vor. Die Reaktionsschicht 14 ist vorzugsweise so dimensioniert, bzw. weist eine derartige Dicke auf, dass der impedanzspektroskopische Erfassungsbereich praktisch vollständig ausgenutzt ist, was bei einer Dicke der Reaktionsschicht von etwa 2 bis 100 µm auf jeden Fall erreicht wird und in der Praxis bereits bei 2 - 10 *µ*m der Fall ist. Bei entsprechender Konzentration von Fängermolekülen 15 in diesem Bereich kann somit der bindungsspezifische Messeffekt des Biochips wesentlich erhöht werden.

Die Reaktionsschicht 14 ist so beschaffen, das sie ein wässriges Reaktionsmedium zur Verfügung stellt. Weiterhin ist sie so beschaffen, dass Zielmoleküle 19 oder auch andere für eine Reaktion benötigte Stoffe, beispielsweise Polymerase, in sie eindiffundieren können, ohne das dabei ihre Aktivität beeinträchtigt wird.

Wie schon oben erwähnt, wird erfindungsgemäß ein Hydrogel als Reaktionsschicht 14 verwendet. Ein Hydrogel stellt ein wässriges Milieu in mechanisch stabiler Form bei gleichzeitiger Gewährleistung des Stoffaustausches in einer überwiegend wässrigen Umgebung dar. Durch Wahl der chemischen Zusammensetzung, was die Komponenten und deren Verhältnis untereinander betrifft, können die Eigenschaften der Hydrogele, wie Wassergehalt, Quellverhalten, mechanische Stabilität, etc. über weite Bereiche variiert werden.

Ein Hydrogel, das leicht herstellbar ist, und das eine gute Haftung sowohl zur Elektrodenanordnung 5 als auch zur Isolierschicht 13 aufweist, ist ein radikalisch vernetzbares Hydrogel auf Acrylamidbasis, das ein Comonomer enthält, welches eine kovalente Ankopplung entsprechend modifizierter Fängermoleküle über Linkergruppen ermöglicht. Das Hydrogel umfasst neben der Monomervorstufe des Polyacrylamids ein Vernetzungsmittel, wenigstens einen Radikalinitiator, wenigstens ein Comonomer mit reaktiven Linkergruppen und gegebenenfalls wenigstens einen Weichmacher. Nach Schichtherstellung und anschließender thermischer bzw. Fotovernetzung wird ein mit Wasser quellbares Hydrogel erhalten, dass-reaktive Linkergruppen zur Immobilisierung von Fängermolekülen enthält. Als Vernetzungsmittel werden Methylenbisacrylamid und/oder Dimethylacrylsäureester, beispielsweise Tetraethylenglykoldimethacrylat eingesetzt.

Durch eine Variation der Konzentrationen des Vernetzungsmittels lässt sich die Maschenweite des Hydrogels einstellen. Das verwendete Comonomer enthält Maleinsäureanhydrid und/oder Glycidyl(meth)acrylat. Als Weichmacher eignet sich Mono-Di- und/oder Triethylenglykol. Die genannten Ausgangsstoffe sind mit einem polaren, mit Wasser mischbaren Lösungsmittel, vorzugsweise mit Dimethylformamid vermengt.

Durch die Variation des Lösungsmittelsanteiles kann die Verarbeitungsviskosität eingestellt werden. Die Haftung an der Flachträgeroberfläche sowie an der Elektrodenanordnung 5 kann durch Beimengung üblicher Haftvermittler beispielsweise auf Silanbasis verstärkt werden.

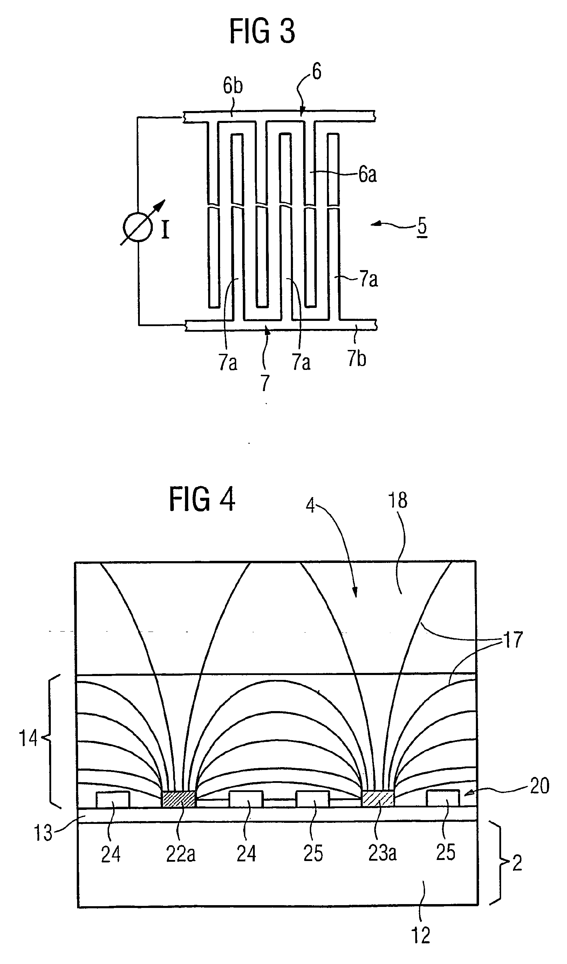

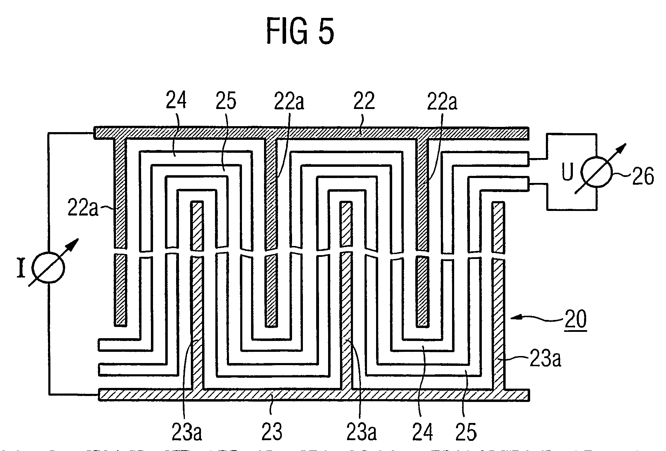

In Fig. 4 und 5 ist ein Ausführungsbeispiel mit einer 4-polaren Elektrodenanordnung 20 dargestellt. Die Elektrodenanordnung 20 setzt sich aus zwei Stromelektroden 22, 23 und zwei Spannungs- bzw. Sondenelektroden 24, 25 zusammen. Die Stromelektroden 22, 23 sind entsprechend der Elektrodenanordnung 5 des Ausführungsbeispiels nach Fig. 2 angeordnet und ausgestaltet. Die Sondenelektroden 24, 25 sind ebenfalls streifenförmig und erstrecken sich als mäanderförmiger Doppelstrang durch die zwischen den Teilelektroden 22a und 23a vorhandenen Zwischenräume hindurch. Die Stromelektroden 22, 23 sind mit einem hochfrequenten Wechselstrom beaufschlagt. An den Sondenelektroden 24, 25 liegt ein Spannungsmesser 26 an, mit dem eine Veränderung des elektrischen Wechselfeldes in Folge von Bindungsereignissen detektierbar ist.

Die Messung kann somit unabhängig von den Stromelektroden erfolgen, so dass sich z.B. deren die Elektrodenimpedanz erhöhende Polarisation nicht auf die Messung auswirken kann. Dagegen muss bei einer 2-poligen Elektrodenanordnung die Elektrodenimpedanz durch eine entsprechend hohe, messtechnisch ungünstige Messfrequenz gering gehalten werden, um den für die Messung ausschlaggebenden Widerstand der Analytlösung bzw. der Reaktionsschicht bestimmen zu können.

Zur Erfassung von durch Bindungsereignisse hervorgerufene Kapazitätsänderungen innerhalb der Reaktionsschicht ist eine 2-polige Elektrodenanordnung in Kombination mit sehr hohen Messfrequenzen (> 1MHz) vorteilhaft.

## Patentansprüche

1. DNA-Chip mit einem Flachträger (2) und einem darauf angeordneten Array (3) von Fängermolekülen enthaltenden Spots(4), wobei jedem Spot (4) eine Mikroelektroden-Anordnung (5) zur Detektion von Bindungsereignissen zwischen den Fängermolekülen und mittels einer Analytlösung applizierten Zielmolekülen zugeordnet ist, wobei die Elektrodenanordnung (5) zumindest teilweise in eine für Zielmoleküle durchlässige hydrophile Reaktionsschicht (14) eingebettet ist, in der immobilisierte Fängermoleküle dreidimensional verteilt sind und wobei die Reaktionsschicht eine Dicke von 2 bis 100 µm aufweist, die etwa im Bereich von 1 bis 5 L liegt, wobei L die Summe aus Elektrodenbreite und Elektrodenabstand ist.

2. DNA-Chip nach Anspruch 1, **dadurch gekennzeichnet, dass** die Elektrodenbreite und der Elektrodenabstand im Bereich von 1 µm (= 1000 nm) liegen und dass die Reaktionsschicht eine Dicke zwischen 2 und 10 µm hat.

3. DNA-Chip nach Anspruch 1, wobei die Mikroelektrodenanordnung ein Zwei-Pol-System ist, **dadurch gekennzeichnet, dass** die Reaktionsschicht eine Dicke von etwa 3 µm hat.

4. DNA-Chip nach Anspruch 1, wobei die Mikroelektrodenanord-nung ein Vier-Pol-System ist, **dadurch gekennzeichnet, dass** die Reaktionsschicht eine Dicke von etwa 7 µm hat.

5. DNA-Chip nach einem der Ansprüche 1 bis 4, **dadurch gekennzeichnet, dass** die Reaktionsschicht (14) bis etwa 95°C thermisch stabil ist.

6. DNA-Chip nach einem der Ansprüche 1 bis 5, **dadurch gekennzeichnet, dass** die Reaktionsschicht (14) Kopplungsgruppen zur kovalenten Bindung von Fängermolekülen enthält.

7. DNA-Chip nach einem der Ansprüche 1 bis 6, **dadurch gekennzeichnet, dass** die Reaktionsschicht (14) ein Hydrogel ist.

8. DNA-Chip nach Anspruch 4, **gekennzeichnet durch** ein radikalisch vernetzbares Hydrogel auf Acrylamidbasis mit Maleinsäureanhydrid und/oder Glycidyl(meth)acrylat als Kopplungsgruppen.

9. DNA-Chip nach einem der Ansprüche 1 bis 8, gekennzeichnet durcheine interdigitale Elektrodenanordnung (5).

10. DNA-Chip nach Anspruch 9, **dadurch gekennzeichnet, dass** die interdigitale Elektrodenanordnung ein Zwei-Pol-Mikroelektrodensystem ist.

11. DNA-Chip nach Anspruch 9, **dadurch gekennzeichnet, dass** die interdigitale Elektrodenanordnung ein Vier-Pol-Mikroelektrodensystem ist.

12. DNA-Chip nach einem der Ansprüche 1 bis 11, **dadurch gekennzeichnet, dass** der Flachträger (2) eine Halbleiterschicht und eine damit verbundene Isolierschicht (13) umfasst, wobei letztere auf ihrer der Halbleiterschicht abgewandten Seite die Elektrodenanordnung (5) und die Reaktionsschicht (14) trägt.

13. DNA-Chip nach Anspruch 12, **dadurch gekennzeichnet, dass** die Halbleiterschicht eine Siliziumschicht (12) ist.

## Claims

1. DNA chip comprising a flat carrier (2) and, arranged along an array (3) of spots (4) containing catcher molecules, each spot (4) being assigned a microelectrode arrangement (5) for detecting binding events between the catcher molecules and target molecules applied by means of an analyte solution, the electrode arrangement (5) being at least partially embedded in a hydrophilic reaction layer (14) which is permeable to target molecules and in which immobilized catcher molecules are distributed three-dimensionally, and the reaction layer having a thickness of 2 to 100 µm, lying approximately in the range of 1 to 5 L, where L is the sum of electrode width and electrode spacing.

2. DNA chip according to Claim 1, **characterized in that** the electrode width and the electrode spacing lie in the region of 1 µm (=̂ 1000 nm) and **in that** the reaction layer has a thickness of between 2 and 10 µm.

3. DNA chip according to Claim 1, the microelectrode arrangement being a two-pole system, **characterized in that** the reaction layer has a thickness of approximately 3 µm.

4. DNA chip according to Claim 1, the microelectrode arrangement being a four-pole system, **characterized in that** the reaction layer has a thickness of approximately 7 µm.

5. DNA chip according to one of Claims 1 to 4, **characterized in that** the reaction layer (14) is thermally stable up to approximately 95°C.

6. DNA chip according to one of Claims 1 to 5, **characterized in that** the reaction layer (14) contains coupling groups for the covalent binding of catcher molecules.

7. DNA chip according to one of Claims 1 to 6, **characterized in that** the reaction layer (14) is a hydrogel.

8. DNA chip according to Claim 4, **characterized by** an acrylamide-based radical-crosslinkable hydrogel with maleic anhydride and/or glycidyl (meth)acrylate as coupling groups.

9. DNA chip according to one of Claims 1 to 8, **characterized by** an interdigital electrode arrangement (5).

10. DNA chip according to Claim 9, **characterized in that** the interdigital electrode arrangement is a two-pole microelectrode system.

11. DNA chip according to Claim 9, **characterized in that** the interdigital electrode arrangement is a four-pole microelectrode system.

12. DNA chip according to one of Claims 1 to 11, **characterized in that** the flat carrier (2) comprises a semiconductor layer and an insulating layer (13) connected thereto, the latter carrying the electrode arrangement (5) and the reaction layer (14) on its side remote from the semiconductor layer.

13. DNA chip according to Claim 12, **characterized in that** the semiconductor layer is a silicon layer (12).

## Revendications

1. Puce à ADN ayant un support ( 2 ) plat et, disposée sur lui, une matrice ( 3 ) de taches ( 4 ) contenant des molécules de capture, dans laquelle à chaque tache ( 4 ) est associé un agencement ( 5 ) de microélectrodes pour la détection d'évènements de liaison entre les molécules de capture et des molécules cibles appliquées au moyen d'une solution d'analyte, l'agencement ( 5 ) d'électrodes étant incorporé, au moins en partie, dans une couche ( 14 ) de réaction hydrophile, perméable aux molécules cibles et dans laquelle des molécules de capture immobilisées sont réparties en trois dimension, la couche de réaction ayant une épaisseur de 2 à 100 µm, qui se trouve à peu près dans la plage de 1 à 5 L, L étant la somme de la largeur d'une électrode et de la distance entre les électrodes.

2. Puce à ADN suivant la revendication 1, **caractérisée en ce que** la largeur d'une électrode et la distance entre les électrodes sont dans la plage de 1 µm ( = 1000 nm ) et **en ce que** la couche de réaction a une épaisseur comprise entre 2 et 10 µm.

3. Puce à ADN suivant la revendication 1, dans laquelle l'agencement de microélectrodes est un système bipolaire, **caractérisée en ce que** la couche de réaction a une épaisseur d'environ 3 µm.

4. Puce à ADN suivant la revendication 1, dans laquelle l'agencement de microélectrodes est un système quadripolaire, **caractérisée en ce que** la couche de réaction a une épaisseur d'environ 7 µm.

5. Puce à ADN suivant l'une des revendications 1 à 4, **caractérisée en ce que** la couche ( 14 ) de réaction est stable thermiquement jusqu'à environ 95°C.

6. Puce à ADN suivant l'une des revendications 1 à 5, **caractérisée en ce que** la couche ( 14 ) de réaction contient des groupes de couplage pour la liaison par covalence de molécules de capture.

7. Puce à ADN suivant l'une des revendications 1 à 6, **caractérisée en ce que** la couche ( 14 ) de réaction est un hydrogel.

8. Puce à ADN suivant la revendication 4, **caractérisée par** un hydrogel réticulable par voie radicalaire à base d'acrylamide avec de l'anhydride maléique et/ou du méthacrylate de glycidyle comme groupes de couplage.

9. Puce à ADN suivant l'une des revendications 1 à 8, **caractérisée par** un agencement ( 5 ) d'électrodes interdigité.

10. Puce à ADN suivant la revendication 9, **caractérisée en ce que** l'agencement d'électrodes interdigitées est un système d'électrodes bipolaires.

11. Puce à ADN suivant la revendication 9, **caractérisée en ce que** l'agencement d'électrodes interdigité est un système de microélectrodes quadripolaires.

12. Puce à ADN suivant l'une des revendications 1 à 11, **caractérisée en ce que** le support ( 2 ) plat comprend une couche semi-conductrice et une couche ( 13 ) isolante qui y est reliée, la couche ( 13 ) isolante portant l'agencement ( 5 ) d'électrodes et la couche ( 14 ) de réaction sur sa face éloignée de la couche semi-conductrice.

13. Puce à ADN suivant la revendication 12, **caractérisée en ce que** la couche semi-conductrice est une couche ( 12 ) de silicium.
